# EUROPEAN PATENT SPECIFICATION

(11) **EP 2 498 827 B1**
(45) Date of publication and mention of the grant of the patent: **16.07.2014**
(21) Application number: 10775832.8
(22) Date of filing: 11.11.2010
(51) Int. Cl.: A61L 9/04, A61L 9/12

(54) **AIR-FRESHENING DEVICE**
LUFTERFRISCHUNGSVORRICHTUNG
DISPOSITIF D'ASSAINISSEMENT D'AIR

(30) Priority: 12.11.2009 IT MI20091982
(43) Date of publication of application: 19.09.2012
(73) Proprietor: Castelberg Technologies Srl, 24064 Grumello Del Monte (BG) (IT)
(72) Inventor: Pagani, Carlo, 24060 Castelli Calepio BG (IT)
(74) Representative: Spina, Alessandro
(86) International application number: PCT/EP2010/067277
(87) International publication number: WO 2011/058093

(56) References cited:
- DE-A1-102007 032 229
- DE-C1- 19 611 993
- US-A1- 2004 121 111
- US-A1- 2006 196 964
- US-B1- 6 264 887

## Description

The present invention relates to an air-freshening device for environments and in particular to an air-freshening device of a passive type.

Air-freshening devices are known for many years for use in environments such as e.g. rooms of a house and compartments of vehicles. Generally speaking, there are air-freshening devices of an active type, which comprise a container for an aerosol substance and wherein the container is provided with a manual or automatic dispenser, and air-freshening devices of a passive type, which are comprised of an air-freshening body impregnated with a scented essence that is diffused in the surrounding environment by convection.

Among the air-freshening devices of the passive type that are presently on the market three main product types may be distinguished. In a first product type the air-freshening bodies are made of cardboard or absorbent paper impregnated with one or more fragrance oils. In a second product type the air-freshening bodies are sticks or wicks that are impregnated by way of capillarity by one or more fragrance oils stored in a container. The air-freshening devices of the second product type are the most diffused ones, because they are considered the most efficient ones from the point of view of the releasing speed of the scented essences. In a third product type the air-freshening bodies are made of containers having a porous matrix and containing powders or ashes scented by means of one or more fragrance oils.

The air-freshening bodies of the passive air-freshening devices may also be advantageously arranged in dispensers provided with suitable housings that have a plurality of venting apertures. In general these apertures can be selectively closed, e.g. by means of adhesive films or mechanically, in order to adjust the flow rate associated with the release of the scented essences. The dispensers may also be provided with electrical resistors and/or electric fans suitable to enhance the release effect of the scented essences.

The known passive air-freshening devices of the above-described types, and in particular those of the second product type, ensure a quick release of the scented essences, which are thus rapidly perceived anywhere in the environment in which the device is present. However, these devices show several drawbacks. In particular, in the air-freshening devices of the first product type, the air-freshening bodies made of cardboard or absorbent paper have an extremely limited service life and, if they are excessively impregnated, they can easily release the fragrance oils, thus soiling the adjacent surfaces. The air-freshening devices of the second product type can be dangerous for the health of the persons and for the objects adjacent thereto in the case the fragrance oils are inadvertently poured from the container or the container is broken. The air-freshening devices of the third product type, which consist in air-freshening bodies containing scented powders and/or ashes, are instead very delicate and must be always handled with care in order to avoid to break them with the consequent dispersion of the fragrance oils or the scented powders and/or ashes in the environment.

A fourth product type of passive air-freshening devices, which is at present less diffused than the previous ones, is also known. In this type of devices the air-freshening body is made of a polymeric material that is impregnated with fragrance oils and injection molded.

Patent US 5820791 discloses, for instance, an example of a device configured for the use in air circulation systems and comprising an air-freshening body made of a polymeric material impregnated with fragrance oils. This body has a prismatic shape and comprises a plurality of through-openings or passages formed in the direction of the thickness. The air-freshening body further comprises a plurality of clamping members arranged along its periphery and suitable to allow to mount it to grids of an air circulation system.

The air-freshening devices of the fourth product type, i.e. those formed of air-freshening bodies made of a polymeric material, are preferable to the air-freshening devices of the first three product types, because they can ensure a longer service life, they are easy to handle, free from the problems concerning the loss of their content in the environment and extremely robust. However, these devices have a low speed of release of the scented essences, which results in a poor olfactory performance, also taking into account the small size an air-freshening device must have. This does not allow to completely release the fragrance oils contained in the air-freshening body and has determined so far their diffusion as air-freshening devices for closed spaces having a small size, e.g. cupboards and drawers, but not as air-freshening devices for environments as an alternative to the air-freshening devices already known and available on the market. In an air-freshening device in fact the releasing speed of the scented essences is considered one of the most important evaluation parameters from the point of view of the final user and thus a parameter having the maximum importance for the manufacturers.

It is therefore an object of the present invention to provide an air-freshening device, which is free from said drawbacks. Said object is achieved with an air-freshening device, whose main features are disclosed in the first claim, while other features are disclosed in the remaining claims.

Thanks to the use of an air-freshening body made of an elastomeric material and having a large surface area with respect to the overall volume, the air-freshening device according to the present invention allows to obtain high releasing speeds of the scented essences, that can be compared to the releasing speeds typical of the known air-freshening devices, and in particular the air-freshening devices of the second product type, as well as to release substantially the whole amount of scented essences.

An advantage provided by the present invention is that the choice of a uniform and small thickness of the air-freshening body allows to remarkably enhance the release effect of the scented essences.

Moreover, the passages formed in the air-freshening body may be advantageously configured to create acceleration effects of the air flow and/or turbulence effects, which are suitable to enhance the convective exchange with the surfaces of the air-freshening body and hence the release effect of the scented essences.

Another advantage provided by the invention is that the air-freshening body may be provided with supporting and/or clamping means integrally formed therewith and suitable to allow to place it on surfaces and/or to couple it to profiles of pieces of furniture, doors, windows, air circulation vents, as well as to other air-freshening bodies, thus allowing in the latter case to obtain several fragrance combinations depending on the preferences and needs of the user.

Further advantages and features of the air-freshening device according to the present invention will become clear to those skilled in the art from the following detailed and non-limiting description of an embodiment thereof with reference to the attached drawings, wherein:
- figure 1 shows a top perspective view of an air-freshening device according to the invention;
- figure 2 shows a cross-section of the air-freshening device according to the invention taken along line II-II of figure 1; and
- figure 3 schematically shows a dispenser comprising the air-freshening device of figure 1.

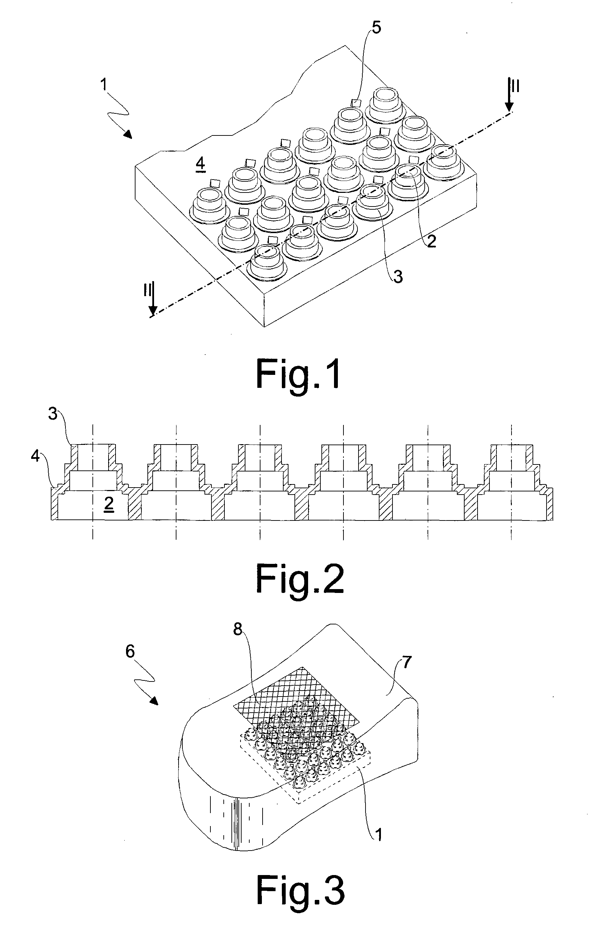

Referring to figure 1, the air-freshening device according to the invention is comprised in a known way of an air-freshening body 1 made of a polymeric material added with one or more fragrance oils. The air-freshening body 1 has, for example, a prismatic shape and is provided with a plurality of passages 2 suitable to allow an air flow therethrough.

According to inventive concept underlying the invention, the polymeric material forming the air-freshening body 1 is of an elastomeric type, for instance PU, EVA or PEBA (Polyether Block Amide), and the passages 2 are formed within a plurality of hollow members 3 protruding from a base plane 4 of the air-freshening body 1. The protruding members 3 may protrude in opposite directions from the base plane 4 and be substantially perpendicular thereto. Alternatively, the protruding members 3 might protrude from the base plane 4 in a single direction only.

Anyway, the surface area of the air-freshening body 1 that can release scented essences is remarkably larger than the surface area of an air-freshening body of a known type made of a polymeric material, which results in a higher speed of release of the scented essences, comparable to the release speed of the air-freshening devices consisting in air-freshening bodies made of cardboard or absorbent paper, or comprising sticks, wicks or scented powders and/or ashes.

It has been experimentally verified that among the different polymeric materials that can be added with scented essences, such as e.g. PE or PP, for this type of application the use of an elastomeric material such as PU or EVA or, preferably, PEBA, is more effective because the amorphous micro-structure of an elastomeric material facilitates the release of the molecules of the scented essences.

The inventor has also found out that in addition to the choice of a suitable material and to the design of an air-freshening body having a large surface area, the release speed of the scented essences may be further increased by making an air-freshening body 1 having a uniformly small thickness.

Still with reference to figure 2, the protruding members 3 have a thickness lower than or equal to the thickness of the base plane 4, whereby the air-freshening body 1 has a substantially uniform thickness. Moreover, the thickness of the base plane 4 and of the protruding members 3 is preferably comprised between 2 and 3 mm, which is an adequate compromise between the manufacturing needs and the need for a quick release of the scented essences. It has also been experimentally verified that by employing larger thicknesses the release speed of the scented essences is greatly reduced, which is absolutely contrary to the aim of the present invention.

As shown in the embodiment of figure 1, the base plane 4 may also comprise a plurality of passages 5 formed in the direction of the thickness outside the protruding members 3, which allows to further increase the surface area of the air-freshening body 1 and thus the release effect of the scented essences.

The protruding members 3 preferably have a frusto-conical shape, which on the one side is useful for the injection molding process and on the other side allows to obtain an acceleration effect of the air-flow crossing the air-freshening body 1.

In the embodiment shown in the drawings, the protruding members 3 have a stepped profile. This feature also contributes to effectively increase the surface area of the air-freshening body 1 by adding a plurality of annular surfaces that are substantially parallel to the base plane 4. In addition to this, the stepped geometry generates turbulence effects in the air flow, which may further facilitate the release of the scented essences.

Still in the aim of maximizing the release of the scented essences, the stepped configuration may advantageously be combined with a coil geometry (not shown) winding around the axis of each protruding member 3, thus being able to create micro-vortexes within the individual passages 2.

Finally, a further increase in the surface area may be obtained by providing the surfaces of the air-freshening body with a plurality of corrugations (not shown).

The elastomeric material used to manufacture the air-freshening body 1 may also be in the form of an expanded material, which results in a micro-porous structure that, when combined with the particular configurations of the above-described passages 2 and surfaces, allows to optimize the release of the scented essences and in particular to release their whole amount from the air-freshening body.

According to a further aspect of the present invention, the air-freshening body 1 may be provided with supporting and/or clamping means (not shown) e.g. integrally formed therewith and suitable to allow to place it on surfaces and/or to directly couple it to profiles of pieces of furniture, doors, windows and air circulation vents.

The air-freshening body 1 may be variously shaped depending on the aesthetic and functional requirements of the air-freshening device. In addition to the prismatic shape of the embodiment shown in figures 1 and 2, the air-freshening body may have any other shape, e.g. cylindrical, oval, a flower shape and the like.

Moreover, as shown in figure 3, the air-freshening body 1 may be so designed to be coupled to a dispenser 6 provided with a shell housing 7 comprising a plurality of venting apertures 8, preferably having an adjustable flow-area, and possibly provided with electrical resistors and/or electric fans (not shown) suitable to enhance the release effect of the scented essences, thus allowing to use the air-freshening body 1 as a disposable cartridge.

It is clear that the above described and illustrated embodiment of the invention is only an example susceptible of numerous variants. For example, the air-freshening body 1 might be designed so as to allow its coupling in series to other air-freshening bodies impregnated with different scented essences, thus allowing to achieve particular combinations of fragrances that may be variously configured by a user.

## Claims

1. An air-freshening device comprised of an air-freshening body (1) made of a polymeric material added with one or more fragrance oils, said air-freshening body (1) having a plurality of passages (2) formed in the direction of its thickness and suitable to allow an air flow therethrough, **characterized in that** said polymeric material is an elastomeric material and **in that** said passages (2) are formed within a plurality of hollow members (3) protruding from a base plane (4) of the air-freshening body (1).

2. An air-freshening device according to the previous claim, **characterized in that** said elastomeric material is PEBA.

3. An air-freshening device according to any of the previous claims, **characterized,in that** said elastomeric material is an expanded elastomeric material.

4. An air-freshening device according to any of the previous claims, **characterized in that** the thickness of the protruding members (3) is lower than or equal to the thickness of the base plane (4).

5. An air-freshening device according to the previous claim, **characterized in that** the thickness of the protruding members (3) and of the base plane (4) is comprised between 2 and 3 mm.

6. An air-freshening device according to any of the previous claims, **characterized by** further comprising passages (5) formed in the base plane (4) externally to the protruding members (3).

7. An air-freshening device according to any of the previous claims, **characterized in that** the protruding members (3) have a frusto-conical shape.

8. An air-freshening device according to the previous claim, **characterized in that** the protruding members (3) have a stepped profile in a longitudinal section.

9. An air-freshening device according to the previous claim, **characterized in that** said stepped profile defines a coil around the axis of each protruding member (3).

10. An air-freshening device according to any of the previous claims, **characterized in that** the surfaces of the air-freshening body (1) have a plurality of corrugations.

## Patentansprüche

1. Lufterfrischungsvorrichtung, bestehend aus einem Lufterfrischungskörper (1) aus einem polymeren Material, ergänzt durch ein oder mehrere Duftöle, wobei der besagte Lufterfrischungskörper (1) eine Mehrzahl von Durchgängen (2) aufweist, welche in Richtung seiner Dicke eingeformt sowie geeignet sind, einen Luftdurchfluss zu ermöglichen, **dadurch gekennzeichnet, dass** das polymere Material ein elastomeres Material ist und dass die besagten Durchgänge (2) innerhalb einer Mehrzahl von hohlen Körpern (3) eingeformt sind, welche von einer Basisebene (4) des Lufterfrischungskörpers (1) hervorstehen.

2. Lufterfrischungsvorrichtung nach dem vorhergehenden Anspruch, **dadurch gekennzeichnet, dass** das elastomere Material PEBA ist.

3. Lufterfrischungsvorrichtung nach einem der vorhergehenden Ansprüche, **dadurch gekennzeichnet, dass** das elastomere Material ein expandiertes elastomeres Material ist.

4. Lufterfrischungsvorrichtung nach einem der vorhergehenden Ansprüche, **dadurch gekennzeichnet, dass** die Dicke der hervorstehenden Teile (3) kleiner als oder gleich der Dicke der Basisebene (4) ist.

5. Lufterfrischungsvorrichtung nach dem vorhergehenden Anspruch, **dadurch gekennzeichnet, dass** die Dicke der hervorstehenden Teile (3) und der Basisebene (4) zwischen 2 und 3 mm liegt.

6. Lufterfrischungsvorrichtung nach einem der vorhergehenden Ansprüche, ferner **gekennzeichnet durch** in die Basisebene (4) extern von den hervorstehenden Teilen (3) eingeformte Durchgänge (5).

7. Lufterfrischungsvorrichtung nach einem der vorhergehenden Ansprüche, **dadurch gekennzeichnet, dass** die hervorstehenden Teile (3) eine kegelstumpfförmige Gestalt aufweisen.

8. Lufterfrischungsvorrichtung nach dem vorhergehenden Anspruch, **dadurch gekennzeichnet, dass** die hervorstehenden Teile (3) ein in der Längsrichtung abgestuftes Profil aufweisen.

9. Lufterfrischungsvorrichtung nach dem vorhergehenden Anspruch, **dadurch gekennzeichnet, dass** das besagte, abgestufte Profil eine Spule um die Achse jedes hervorstehenden Teils (3) bildet.

10. Lufterfrischungsvorrichtung nach einem der vorhergehenden Ansprüche, **dadurch gekennzeichnet, dass** die Oberflächen des Lufterfrischungskörpers (1) eine Mehrzahl von Wellungen aufweisen.

## Revendications

1. Dispositif de désodorisation composé d'un corps de désodorisation (1) réalisé avec un matériau polymère additionné d'une ou de plusieurs huiles parfumées, ledit corps de désodorisation (1) ayant une pluralité de passages (2) formés dans la direction de son épaisseur et appropriés pour permettre un écoulement d'air à travers ces derniers, **caractérisé en ce que** ledit matériau polymère est un matériau élastomère et **en ce que** lesdits passages (2) sont formés à l'intérieur d'une pluralité d'éléments creux (3) faisant saillie d'un plan de base (4) du corps de désodorisation (1).

2. Dispositif de désodorisation selon la revendication précédente, **caractérisé en ce que** ledit matériau élastomère est le PEBA.

3. Dispositif de désodorisation selon l'une quelconque des revendications précédentes, **caractérisé en ce que** ledit matériau élastomère est un matériau élastomère expansé.

4. Dispositif de désodorisation selon l'une quelconque des revendications précédentes, **caractérisé en ce que** l'épaisseur des éléments en saillie (3) est inférieure ou égale à l'épaisseur du plan de base (4).

5. Dispositif de désodorisation selon la revendication précédente, **caractérisé en ce que** l'épaisseur des éléments en saillie (3) et du plan de base (4) est comprise entre 2 et 3 mm.

6. Dispositif de désodorisation selon l'une quelconque des revendications précédentes, **caractérisé en ce qu'**il comprend en outre des passages (5) formés dans le plan de base (4) extérieurement aux éléments en saillie (3).

7. Dispositif de désodorisation selon l'une quelconque des revendications précédentes, **caractérisé en ce que** les éléments en saillie (3) ont une forme tronconique.

8. Dispositif de désodorisation selon la revendication précédente, **caractérisé en ce que** les éléments en saillie (3) ont un profil étagé dans une section longitudinale.

9. Dispositif de désodorisation selon la revendication précédente, **caractérisé en ce que** ledit profil étagé définit une bobine autour de l'axe de chaque élément en saillie (3).

10. Dispositif de désodorisation selon l'une quelconque des revendications précédentes, **caractérisé en ce que** les surfaces du corps de désodorisation (1) ont une pluralité d'ondulations.
